# EUROPEAN PATENT APPLICATION

(11) **EP 1 736 204 A1**
(43) Date of publication of application: **27.12.2006**
(21) Application number: 05013576.3
(22) Date of filing: 23.06.2005
(51) Int. Cl.: A61N 5/06

(54) **Phototherapy apparatus with the function of change-over to different wavelength**

(71) Applicant: Hsu, Fu-Yu, Taiwan (CN); Hsu, Fu-Shou, Taiwan (CN); Hsu, Fu-Jung, Taiwan (TW)
(72) Inventor: Hsu, Fu-Yu, Taiwan (CN); Hsu, Fu-Shou, Taiwan (CN); Hsu, Fu-Jung, Taiwan (TW)
(74) Representative: Casalonga, Axel

(57) **Abstract**

A phototherapy apparatus with the function of change-over to different wavelength including a housing, a radiation head, and a plurality of light-emitting diodes. The radiation head and a translucent cap are formed in a non-circular contour, and the light-emitting diodes are divided into at least two groups having different wavelength, and two groups of the light-emitting diodes are arranged in a staggered manner on the circuit board. A light-emitting diode (LED) control circuit is disposed within the receiving chamber to supply power to the circuit board and to enable the light-emitting action of one group of the light-emitting diodes on the circuit board. An operating interface is mounted on the surface of the housing and permits a free choice of the groups of the light-emitting diodes for a phototherapy.

## Description

The invention relates to a phototherapy apparatus with the function of change-over to different wavelength, and more particularly to a phototherapy apparatus that can be changed over to different wavelength for treatment. Meanwhile, a well-distributed radiation is ensured to avoid the formation of shadow areas due to the absence of radiation.

With the change of life style, dermal problems have recently focused on the skin disease caused by job stress, dietetic habit or other factors. Hence, it becomes gradually popular to employ Intense Pulsed Light (IPL) to cure the dermal diseases.

The research of Intense Pulsed Light (IPL) began in the year of 1990. The action spectrum of the Intense Pulsed Light (IPL) is very wide und it is the light whose wavelength ranges from 550nm to 1200nm. The spectrum of visible light and the infrared light are also included in the spectrum of the Intense Pulsed Light. Compared with the laser beam, the Intense Pulsed Light has the similar energy and wavelength, but the range of its wavelength is larger. Because the skin tissue has different reaction to the absorption and dispersion of different light beams, the system of Intense Pulsed Light (IPL) can select the relevant light to cure the different dermal problems. For example, the light with color from yellow to orange can exert its effect on the red blood vessel in the skin and the red light can remove the pigment.

For the above mentioned characteristics, aiming to the removal of the dermal problems, the Intense Pulsed Light (IPL) can select an arbitrary light with suitable wavelength to carry out the treatment for different dermal problem, such as the expansion of blood vessel (redness-removal), the pigment treatment (spot-removal) and the stimulation of the fabric root cell in the derma (younger treatment) etc. Besides, the treatment results of spots, the uneven pigment, the expansion of blood vessel, the reddish face, the rough skin without flexibility, etc. are also very satisfactory.

Unfortunately, the instrument of the Intense Pulsed Light (IPL) is very expensive and the operation requirements of this instrument are also very high. Hence, a normal little clinic or family can not afford it. Therefore, a portable light-treating instrument in small size comes into being.

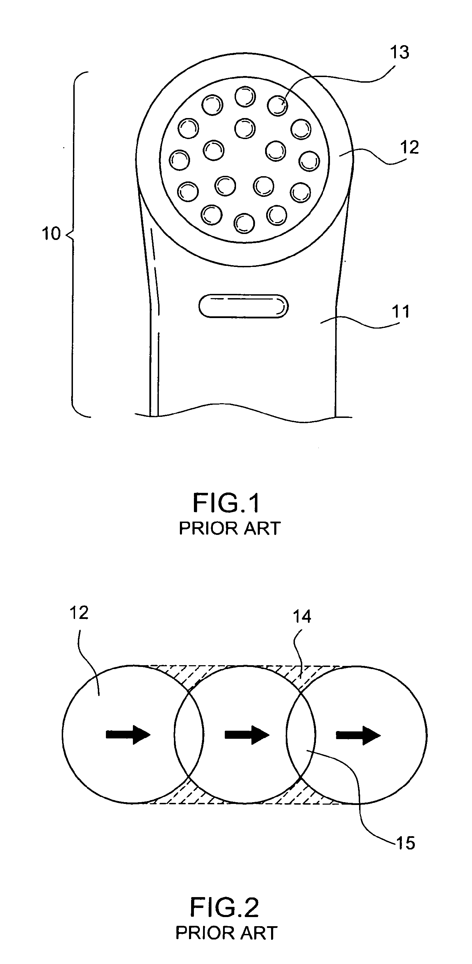

As shown in FIG. 1, a conventional phototherapy apparatus has a housing 11 on which a radiation head 12 is mounted. A plurality of light-emitting diodes 13 with a preset wavelength is installed in the radiation head 12. In use, hold the housing 11 to approach to the skin for carrying out a phototherapy with the preset wavelength. However, the light-emitting diodes 13 can only carry out a single function since they have only one wavelength. Thus, the treatment can't be adjusted to different skin quality and treatment regions. This is one of the drawbacks of the conventional phototherapy apparatus. Moreover, the conventional radiation head 12 has a circular contour. So, a plurality of shadow areas 14 due to the absence of radiation marked by dashed line will be created (see FIG. 2). To reduce the radiation shadow areas 14 as much as possible, will, however, result in intersection areas 15 that would make the local skin reddish and swelling.

It is a primary object of the invention to eliminate the above-mentioned drawbacks and to provide a phototherapy apparatus that includes the function of change-over to different wavelength for a multifunctional application.

It is another object of the invention to provide a multifunctional phototherapy apparatus whose radiation head has a rectangular, rather than a non-circular contour so that the radiation can be well-distributed on the skin so as to reduce shadow areas 14 due to the absence of radiation and to avoid intersection areas during phototherapy. In this way, a better treatment effect of the phototherapy can be ensured.

In order to achieve the above-mentioned objects, a phototherapy apparatus with the function of change-over to different wavelength comprises:
a) a housing adapted to permit an ergonomic holding of the apparatus, a receiving chamber being formed within the housing;
b) a radiation head mounted on the housing, the radiation head having a translucent cap; and
c) a plurality of light-emitting diodes electrically coupled to a circuit board, the circuit board being positioned within the radiation head to enable the radiation of the light-emitting diodes in direction to the translucent cap;
wherein the radiation head and the translucent cap are formed in a non-circular contour, and the light-emitting diodes are divided into at least two groups having different wavelength, and two groups of the light-emitting diodes are arranged in a staggered manner on the circuit board;
wherein an light-emitting diode (LED) control circuit is disposed within the receiving chamber to supply power to the circuit board and to enable the light-emitting action of one group of the light-emitting diodes on the circuit board; and
wherein an operating interface is mounted on the surface of the housing and permits a free choice of the groups of the light-emitting diodes for a phototherapy.

The phototherapy apparatus in accordance with the invention further comprises a light cone that is attached with a mounting portion thereof onto the radiation head. The light cone is tapered from the mounting portion to a light exit that corresponds to the human nose in size. In this way, the phototherapy apparatus in accordance with the invention can be used as a BioNase.

The accomplishment of this and other objects of the invention will become apparent from the following descriptions and its accompanying figures of which:
FIG. 1 is a front view of a conventional phototherapy apparatus;
FIG. 2 is a schematic drawing of the radiation of the conventional phototherapy apparatus in accordance with FIG. 1;
FIG. 3 is a front view of a preferred embodiment of the invention;
FIG. 4 is a side view of the preferred embodiment of the invention in accordance with FIG. 3;
FIG. 5 is a schematic drawing of an arrangement of light-emitting diodes of the invention;
FIG. 6 is a schematic drawing of another arrangement of the light-emitting diodes of the invention;
FIG. 7 is a cutaway view of an assembly structure of a light-emitting diode of the invention;
FIG. 8 is a cutaway view of another assembly structure of a light-emitting diode of the invention;
FIG. 9 is a block diagram of a control circuit of the invention;
FIG. 10 is a schematic drawing of the application of the preferred embodiment of the invention to a facial skin;
FIG. 11 is a side view of a second embodiment of the invention; and
FIG. 12 is a schematic drawing of the application of the second embodiment to a human nose.

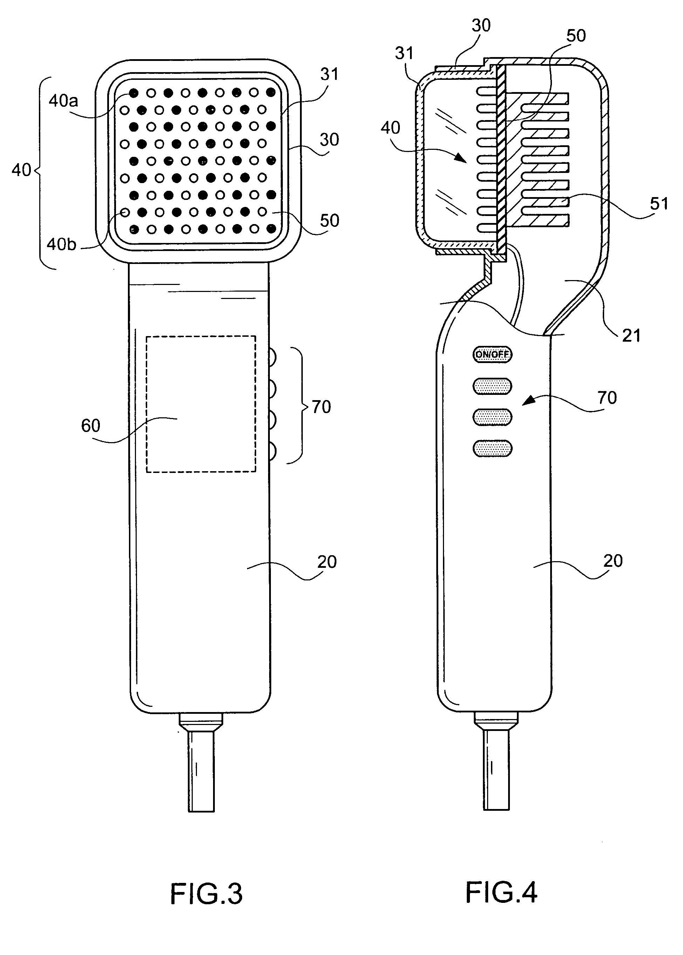

First of all, referring to FIGS. 3 and 4, an embodiment of a phototherapy apparatus in accordance with the invention includes a housing 20 that permit an ergonomic holding of the apparatus, and a radiation head 30 that is disposed on a top side of the housing 20. It's preferable that the housing 20 and the radiation head 30 are integrally formed, but should not be restricted thereto. Alternatively, the radiation head 30 may be individually formed and then attached to the housing 20. The radiation head 30 includes a translucent cap 31 that is put onto the radiation head 30 from inside to outside, but should not be restricted thereto. Alternatively, the translucent cap 31 may be mounted on the radiation head 30 from outside to inside. Besides, the translucent cap 31 may be attached to the radiation head 30 by use of the high frequency processing. This belongs to the prior art so that no further descriptions thereto are given hereinafter.

A plurality of light-emitting diodes 40 is electrically coupled to a circuit board 50 that is disposed within the radiation head 30. The circuit board 50 enables the light-emitting diodes 40 to emit light beams in direction to the translucent cap 31.

Unlike the conventional phototherapy apparatus that has a circular contour, the radiation head 30 and the translucent cap 31 in accordance with the invention are formed with a rectangular contour. Meanwhile, the four corners of thereof can be chamfered, but should not restricted thereto. The rectangular design of the invention can avoid the formation of the shadow areas 14 due to the absence of radiation (see FIG. 2) and ensure a well-distributed radiation effect with more light-emitting diodes 40 on the circuit board 50.

Moreover, the invention features that the light-emitting diodes 40 consists of light-emitting diodes 40a, 40b having different wavelength. Meanwhile, the light-emitting diodes 40a, 40b are arranged in a staggered manner on the circuit board 50.

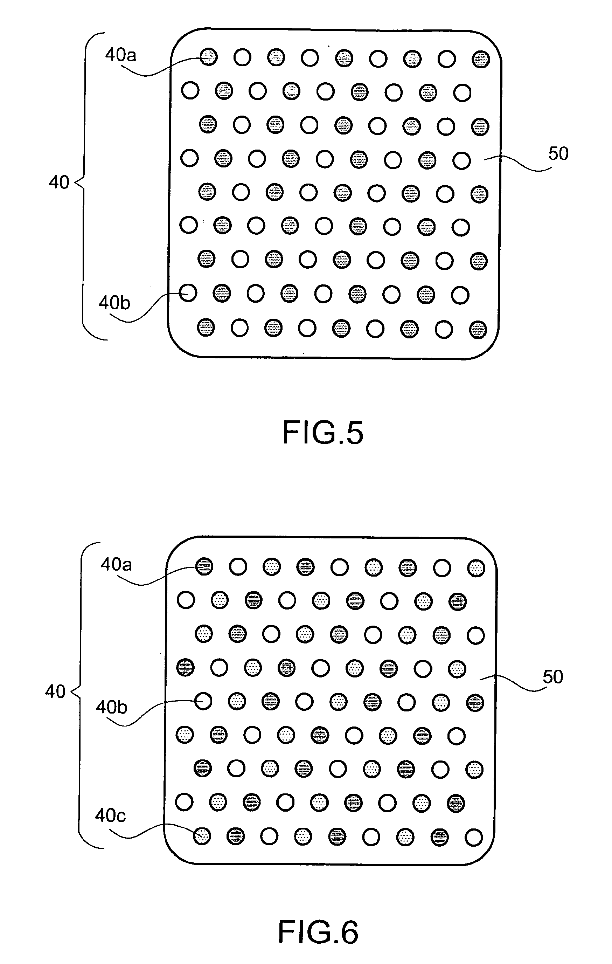

As shown in FIG. 5, the light-emitting diodes 40 include the light-emitting diodes 40a with a first wavelength and the light-emitting diodes 40b with a second wavelength. As shown in FIG. 6, the light-emitting diodes 40 include the light-emitting diodes 40a with a first wavelength, the light-emitting diodes 40b with a second wavelength and the light-emitting diodes 40c with a third wavelength. In taking account of the circuit board area and the circuit layout, the third embodiment of the light-emitting diodes 40a, 40b, 40c with three kinds of wavelength is preferable, but should not be restricted thereto. The staggered arrangement of the light-emitting diodes with different wavelength can be done in such a way that each individual light-emitting diode or each row of the light-emitting diodes is offset from the other under the condition that the light-emitting diodes 40 of each kind of wavelength achieve a sufficient and well-distributed radiation effect.

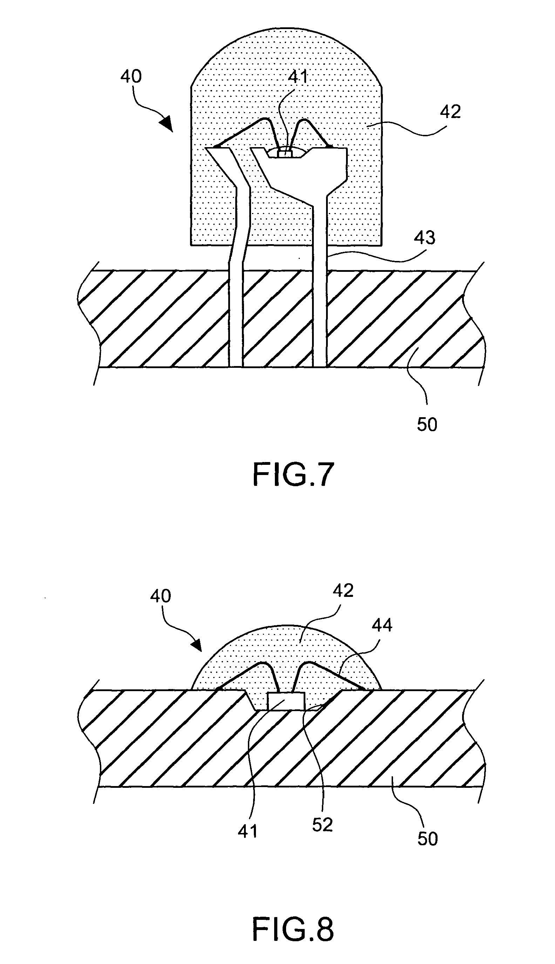

As shown in FIG. 7, the light-emitting diode 40 is made in such a way that LED die 41 is packaged by translucent colloid 42 and electrically coupled to the circuit board 50 via connecting feet 43. Alternatively, the light-emitting diodes 40, as shown in FIG. 8, the LED die 41 is stuck to a recess 52 formed on the surface of the circuit board 50. Then, the LED die 41 is wire-bonded to the circuit board 50 und packaged by the translucent colloid 42 in place. The recess 52 serves as a reflection surface with which the radiation beam can be reflected upwards.

The two above-mentioned assembly ways of the light-emitting diodes 40 and the circuit board 50 are applicable. In order to light up most of the light-emitting diodes 40 with certain wavelength, a plurality of lead is formed on the circuit board by use of the etching technique for the electrical connection. This belongs to the prior art in the field of printed circuit board so that no further description thereto are given hereinafter. In addition, the circuit board 50 includes a plurality of cooing ribs 51 at a bottom surface thereof to enhance the cooling effect of the circuit board 50. Alternatively, the cooing ribs 51 may be replaced by other cooling apparatuses.

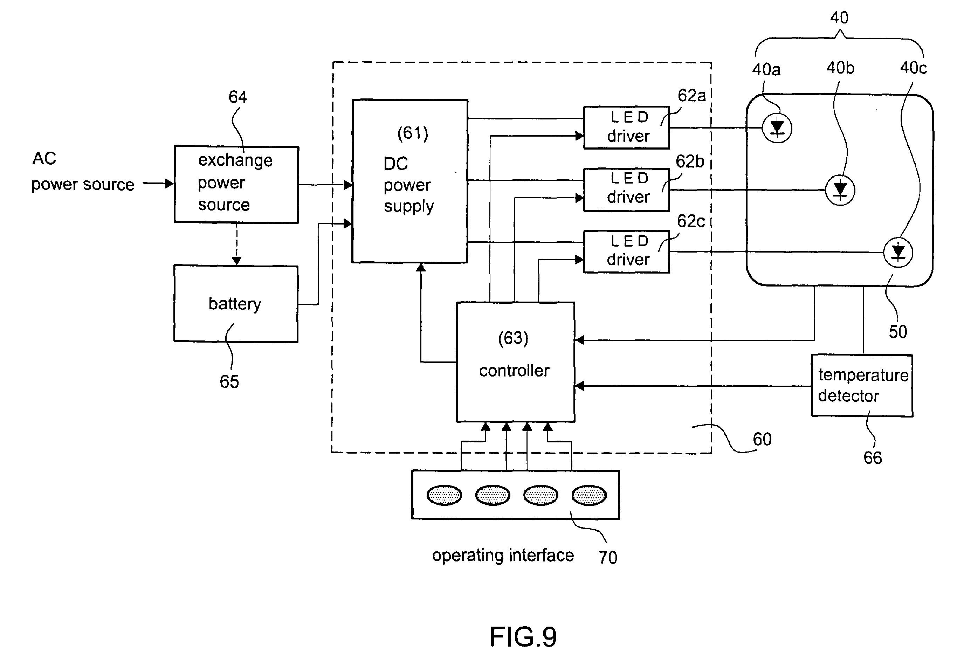

As shown in FIG. 3 and 9, an LED control circuit 60 is disposed within a receiving chamber 21 to supply power to the circuit board 50 and to enable the light-emitting action of one group of the light-emitting diodes 40a, 40b, 40c on the circuit board 50. The LED control circuit 60 includes a DC power supply 61, a plurality of LED driver 62a, 62b, 62c, and a controller 63.

The DC power supply 61 is adapted to supply power to the light-emitting diodes 40 on the circuit board 50. The DC power is created by an exchange power source 64 that converts an alternating current into 1.5 V - 4.5V direct current. The DC power may be supplied by a battery 65 within the housing 20. The battery 65 can be replaced by a secondary battery that is recharged by the exchange power source 64 to supply the DC power.

The LED driver 62a, 62b, 62c are interposed between the DC power supply 61 and the circuit board 50 to put the light-emitting diodes 40a, 40b, 40c in operation, respectively.

The controller 63 is coupled to an operating interface 70 and adapted to control the LED driver 62a, 62b, 62c to activate the light-emitting diodes 40a, 40b, 40c, respectively.

Moreover, a temperature detector 66 is interposed between the controller 63 of the LED control circuit 60 and the circuit board 50 for monitoring the temperature of the light-emitting diodes 40 when they light up, thereby preventing the overtemperature from damaging the human skin. When the temperature of the light-emitting diodes 40 exceeds a predetermined temperature, the controller 63 closes all of the light-emitting diodes 40 for ensuring a better safety in use.

As shown in FIG. 4, the operating interface 70 is mounted on the surface of the housing 20 and adapted to permit a free choice of one group of the light-emitting diodes 40a, 40b, 40c. The operating interface 70 includes a plurality of push buttons, but should not be restricted thereto. Alternatively, the operating interface 70 can be configured as a change-over type operating interface.

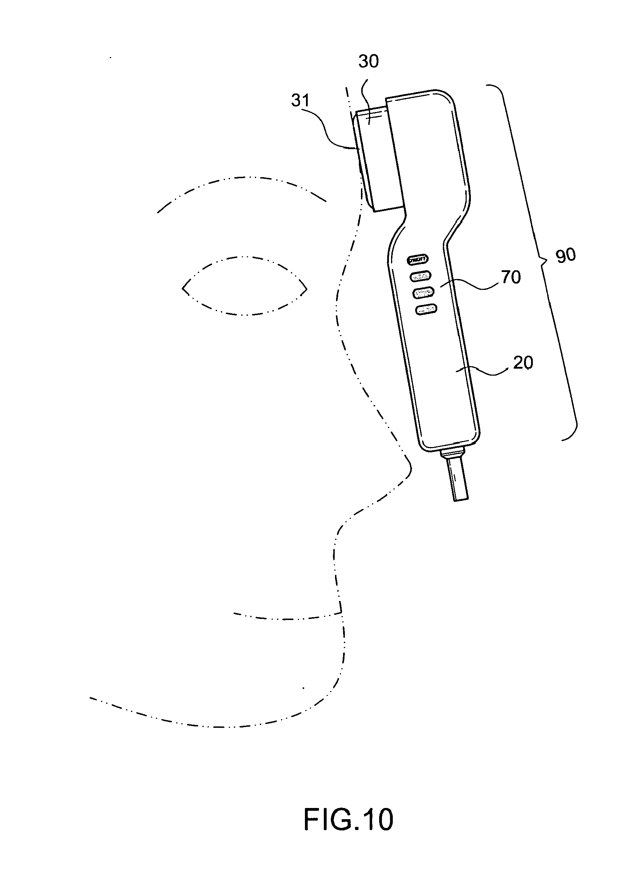

Based upon the above-mentioned technique of the invention, at least three light-emitting diodes of different wavelength are available, thereby creating an application example of a multifunctional phototherapy apparatus as shown in FIG. 10. In use, the operator holds the housing 20 with his hand and depresses one of the push buttons on the operating interface 70 to choose a radiation light beam with desired wavelength to apply to the skin. For example, the first wavelength 300 - 330 nm for ultraviolet rays, the second one is 630 - 660 um for infrared rays, and the third one is 860 um for invisible rays. Of course, a fourth wavelength of 470 nm for blue rays can be added to the invention. This won't be more described hereinafter. Each light wave has its own function, and a suitable one should be chosen by a professional physician. This doesn't belong to the object of the invention so that no further descriptions are given hereinafter.

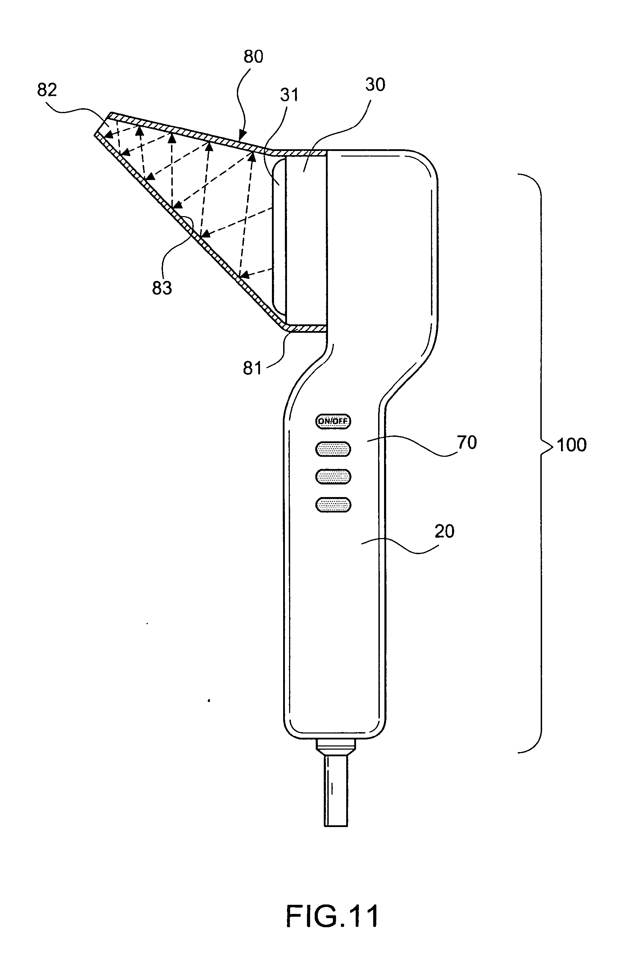

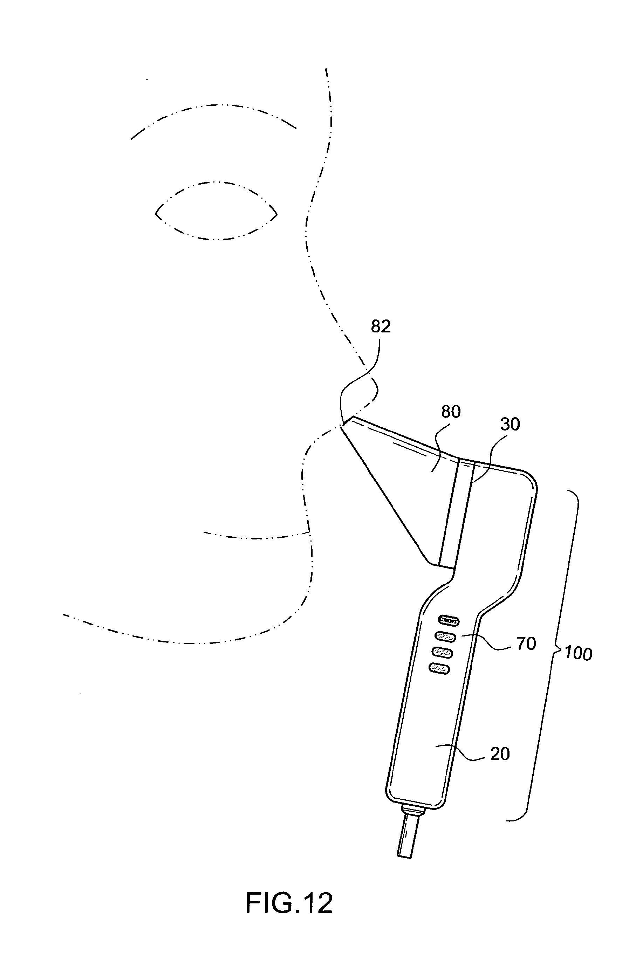

As shown in FIG. 11, a light cone 80 is added to the phototherapy apparatus. The light cone 80 includes a mounting portion 81 that is mounted on the radiation head 30. The light cone 80 is tapered from the mounting portion 81 to a light exit 82. The inner wall of the light cone 80 is preferably constructed as a reflector 83. In this way, a zigzag reflection of the light beams from the translucent cap 31 to the light exit 82 by means of the reflector 83 is ensured to allow for an enhanced radiation effect. Accordingly, the phototherapy apparatus in accordance with the embodiment, as shown in FIG. 12, can serve as a BioNase 100 that features continuous relief from nasal congestion, runny nose, sneezing, itching and teary eyes without any known side-effects. By removing the light cone 80, the invention is returned to be a multifunctional phototherapy apparatus 90 as shown in FIG. 10.

Accordingly, the phototherapy apparatus in accordance with the invention provides a plurality of radiation light beams with different wavelength that can be freely chosen by operators. In addition, the phototherapy apparatus can be used as a BioNase 100 to achieve the multifunctional effect.

Many changes and modifications in the above-described embodiments of the invention can, of course, be carried out without departing from the scope thereof. Accordingly, to promote the progress in science and the useful arts, the invention is disclosed and is intended to be limited only by the scope of the appended claims.

## Claims

1. A phototherapy apparatus with the function of change-over to different wavelength, comprising:
a) a housing adapted to permit an ergonomic holding of the apparatus, a receiving chamber being formed within the housing;
b) a radiation head mounted on the housing, the radiation head having a translucent cap; and
c) a plurality of light-emitting diodes electrically coupled to a circuit board, the circuit board being positioned within the radiation head to enable the radiation of the light-emitting diodes in direction to the translucent cap;
wherein the radiation head and the translucent cap are formed in a non-circular contour, and the light-emitting diodes are divided into at least two groups having different wavelength, and two groups of the light-emitting diodes are arranged in a staggered manner on the circuit board;
wherein an light-emitting diode (LED) control circuit is disposed within the receiving chamber to supply power to the circuit board and to enable the light-emitting action of one group of the light-emitting diodes on the circuit board; and
wherein an operating interface is mounted on the surface of the housing and permits a free choice of the groups of the light-emitting diodes for a phototherapy.

2. The phototherapy apparatus with the function of change-over to different wavelength as recited in claim 1 wherein the light-emitting diode is packaged by translucent colloid and electrically coupled to the circuit board via connecting feet.

3. The phototherapy apparatus with the function of change-over to different wavelength as recited in claim 1 wherein the light-emitting diode in the form of an LED die is stuck to a recess formed on the surface of the circuit board, and the LED die is then wire-bonded to the circuit board und packaged by translucent colloid in place.

4. The phototherapy apparatus with the function of change-over to different wavelength as recited in claim 1 wherein a plurality of cooing ribs is formed at a bottom end of the circuit board.

5. The phototherapy apparatus with the function of change-over to different wavelength as recited in claim 1 wherein the LED control circuit includes:
a) a DC power supply adapted to supply DC power to the light-emitting diodes on the circuit board;
b) a plurality of LED drivers interposed between the DC power supply and the circuit board to put one group of the light-emitting diodes in operation, respectively; and
c) a controller coupled to an operating interface and adapted to control the LED drivers to activate the light-emitting diodes, respectively.

6. The phototherapy apparatus with the function of change-over to different wavelength as recited in claim 5 wherein the DC power is created by an exchange power source that converts an alternating current into 1.5 V - 4.5V direct current.

7. The phototherapy apparatus with the function of change-over to different wavelength as recited in claim 5 wherein the DC power may be supplied by a battery within the housing.

8. The phototherapy apparatus with the function of change-over to different wavelength as recited in claim 5 wherein a temperature detector is interposed between the controller of the LED control circuit and the circuit board for monitoring the temperature of the light-emitting diodes when they light up.

9. A phototherapy apparatus with the function of change-over to different wavelength, comprising:
a) a housing adapted to permit an ergonomic holding of the apparatus, a receiving chamber being formed within the housing;
b) a radiation head mounted on the housing, the radiation head having a translucent cap; and
c) a plurality of light-emitting diodes electrically coupled to a circuit board, the circuit board being positioned within the radiation head to enable the radiation of the light-emitting diodes in direction to the translucent cap;
wherein the radiation head and the translucent cap are formed in a non-circular contour, and the light-emitting diodes are divided into at least two groups having different wavelength, and two groups of the light-emitting diodes are arranged in a staggered manner on the circuit board;
wherein a light-emitting diode (LED) control circuit is disposed within the receiving chamber to supply power to the circuit board and to enable the light-emitting action of one group of the light-emitting diodes on the circuit board;
wherein an operating interface is mounted on the surface of the housing and permits a free choice of the groups of the light-emitting diodes for a phototherapy; and
wherein a light cone is attached with a mounting portion thereof onto the radiation head, and the light cone is tapered from the mounting portion to a light exit, and the light exit corresponds to the human nose in size.

10. The phototherapy apparatus with the function of change-over to different wavelength as recited in claim 8 wherein the inner wall of the light cone is constructed as a reflector.
